# EUROPÄISCHE PATENTSCHRIFT

(11) **EP 0 748 634 B1**
(45) Veröffentlichungstag und Bekanntmachung des Hinweises auf die Patenterteilung: **16.04.2003**
(21) Anmeldenummer: 96109297.0
(22) Anmeldetag: 11.06.1996
(51) Int. Cl.: A61L 27/00

(54) **Implantat, seine Verwendung in der Chirurgie und Verfahren zu seiner Herstellung**
Implant, its use in surgery and method for its manufacture
Implant, son utilisation en chirurgie et son procédé de fabrication

(30) Priorität: 14.06.1995 DE 19521642
(43) Veröffentlichungstag der Anmeldung: 18.12.1996
(73) Patentinhaber: Aesculap AG & Co. KG, 78532 Tuttlingen (DE)
(72) Erfinder: Goldmann, Helmut, Dr., 34212 Melsungen (DE)
(74) Vertreter: Patentanwälte Ruff, Wilhelm, Beier, Dauster & Partner

(56) Entgegenhaltungen:
- EP-A- 0 087 662
- DE-A- 3 913 926
- GB-A- 2 153 235
- US-A- 5 268 178

## Beschreibung

Die vorliegende Erfindung betrifft ein Implantat zur Verwendung in der Chirurgie und Verfahren zu seiner Herstellung.

Infektionen nach einem chirurgischen Eingriff stellen insbesondere in der Gefäßchirurgie eine akute Gefahr für das Leben des Patienten dar und führen häufig zur Amputation der behandelten Extremität. Das Problem der Protheseninfektion wird in der medizinischen Fachliteratur ausführlich diskutiert.

Bei der systemischen Verabreichung von Antibiotika zum Erzielen eines Infektionsschutzes zeigen sich Schwierigkeiten bei der Einstellung eines optimalen Serumspiegels, Risiken wie Resistenzbildung und für den Patienten belastende Nebenwirkungen. Es wurden daher Implantate entwickelt, die mit antimikrobiellen Wirkstoffen wie beispielsweise bakteriziden Schwermetallen oder Antibiotika versehen sind.

Neuere Entwicklungen umfassen die Modifizierung von Gefäßprothesenmaterial mit Antibiotika zur Infektionsprophylaxe.

In der Patentschrift US 44 42 133 wird ein Verfahren zur Herstellung einer Gefäßprothese beschrieben, an deren Oberfläche ein Antibiotikum gebunden ist. Die Oberfläche der Prothese, aus PTFE oder Polyethylenterephthalat, wird zunächst mit einer wäßrigen Lösung aus Tridodecylmethylammoniumchlorid (TDMA) oder Benzalkoniumchlorid behandelt. Nach dieser Aktivierung der Prothesenoberfläche mit dem positiv geladenen Ankermolekül wird im zweiten Verfahrensschritt ein anionisches Antibiotikum wie Penicillin oder Oxicillin durch erneute Tauchung der Prothese über die Ankergruppe an die Prothesenoberfläche gebunden.

Die Gefahr, dass mit dem Antibiotikum nicht abgesättigte, oberflächenaktive Ankergruppen blutschädigend und thrombogen wirken, wird in der US 47 40 382 dadurch beseitigt, dass freie, nicht besetzte Ankermoleküle mit einem Kationenaustauscher Sepharose-CM oder Sulphopropylcellulose von der Prothesenoberfläche wieder entfernt werden.

Der Nachteil, dass mit den bisher beschriebenen Verfahren lediglich negativ geladene Antibiotika an die Prothesenoberfläche gebunden werden können, wird in der US 47 49 585 dadurch beseitigt, dass über negativ geladenen oberflächenaktive Ankermoleküle, wie z. B. Alkylacrylsulfonate, kationische Antibiotika die Prothesenoberfläche besetzen können. Der weitere Nachteil, dass nur ladungstragende Antibiotika nach den Verfahren verwendet werden können und die Abgaberate des Antibiotikums nicht kontrolliert werden kann, wird dadurch beseitigt, dass an die kationisch aktivierte Prothesenoberfläche negativ geladene Phospholipidvesikel (Liposomen), die das Antibiotikum eingekapselt enthalten, gebunden werden. Das Verfahren ist ebenfalls bei einer negativ geladenen Oberfläche und einem positiv geladenen Liposom anwendbar.

Weitere Verfahren zur Herstellung infektionsresistenter Implantate werden von Fox und Modark beschrieben in US 4563485, US 4581028, US 4612337 und EP 207624. Diese Verfahren beruhen auf der mehrfachen Durchtränkung der Implantate mit antibiotikahaltigen Lösungen aus der Klasse der Chinoline oder Sulfonamide und Bindung des Antibiotikums in Form des Silbersalzes an die Implantatoberfläche.

EP 328421 beschreibt die Herstellung infektionsresistenter Implantatoberflächen durch Verwendung eines matrixbildenden polymeren Materials und eines Silbersalzes eines Biguanids (spez. Chlorhexidin), indem das Polymer und das antimikrobielle Mittel zu einer Beschichtungszusammensetzung zusammengemischt werden, mit der dann die Implantatoberfläche beschichtet werden kann.

Antibiotika-Kollagenformulierungen zur Beschichtung von porösen Trägern, mit dem Ziel, eine anhaltende Freigabe des Wirkstoffes aus dem Implantat nach Implantation zu ermöglichen, sind ebenfalls beschrieben in DE 3503126.

Die beschriebenen Verfahren haben erhebliche Nachteile. So sind sie zum Teil technisch sehr aufwendig und zeit- und arbeitsintensiv, da verschiedene Schritte mehrfach durchlaufen werden müssen. Teilweise ist eine direkte Vorbereitung im OP notwendig. Unerwünschte Effekte wie toxische Reaktionen und erhöhte Thrombogenität sind möglich. Weiterhin kann es durch Nachbehandlungsschritte wie Vernetzung einer Antibiotika enthaltenden Kollagenbeschichtung zu unerwünschten Nebenreaktionen des Vernetzungsmittels mit dem Antibiotikum kommen.

Es stellte sich daher die Aufgabe, ein Implantat mit antimikrobiotischer Wirkung zur Verfügung zu stellen, das sicher und zuverlässig zum Vorteil des Patienten anzuwenden ist sowie nach einfachen Verfahrensweisen kostengünstig herzustellen ist.

Diese Aufgabe wird gelöst durch ein Implantat, das mindestens teilweise und mindestens an den infektionsgeschützten Oberflächenbezirken aus resorbierbarem Material besteht und in diesem einen antimikrobiotischen Wirkstoff enthält, einen nicht resorbierbaren Grundkörper aufweist, der mindestens eine den antimikrobiotischen Wirkstoff enthaltende Beschichtung oder Imprägnierung aufweist, die aus mindestens einem antimikrobiotischen Wirkstoff und mindestens einem resorbierbaren Material gebildet ist, dadurch gekennzeichnet, dass der antimikrobiotische Wirkstoff in Wasser schwerlöslich ist, insbesondere eine Löslichkeit in Wasser von weniger als 10 mg/ml aufweist, und die Löslichkeit des antimikrobiotischen Wirkstoffes so gewählt ist, dass er im wesentlichen während der gesamten Degradationszeit des Materials abgegeben wird, wobei das resorbierbare Material ausgewählt ist aus Polylactiden, Polyglykoliden, Copolymeren von diesen, Glykolid-Trimethylencarbonatcopolymeren, Copolymeren mit anderen resorbierbaren cyclischen Estern oder Dioxanonen sowie Terpolymeren davon.

Das erfindungsgemäße Implantat kann insbesondere so ausgebildet sein, dass die Löslichkeit des antimikrobiotischen Wirkstoffs und die Degradationszeit des resorbierbaren Materials so aufeinander abgestimmt sind, dass der antimikrobiotische Wirkstoff während der Degradationszeit des Materials in abnehmenden Mengen abgegeben wird. Mit Vorteil kann sich die Freisetzungskinetik des antimikrobiotischen Wirkstoffes durch einen Retardeffekt bei der Wirkstoffabgabe auszeichnen. Die Freisetzung des Wirkstoffs erfolgt nach einem Diffusionsmechanismus. Auf diese Weise kann die Hauptmenge des antimikrobiotischen Wirkstoffes in den ersten Tagen nach dem Eingriff, wo die Infektionsgefahr am größten ist, abgegeben werden. In der Anfangsphase kann die Wirkstoffabgabe in im wesentlichen kontinuierlich abnehmenden Mengen erfolgen. Durch die gleichzeitige Degradation des den antimikrobiotischen Wirkstoff enthaltenden resorbierbaren Materials nimmt die vom Wirkstoff zu überwindende Diffusionsstrecke ab, was einer verminderten Wirkstoffabgabe infolge der Konzentrationsverringerung im resorbierbaren Material entgegenwirkt.

Das Implantat gemäß der vorliegenden Erfindung kann vorteilhaft so ausgebildet sein, dass es einen nichtresorbierbaren Grundkörper aufweist, der mindestens eine den antimikrobiotischen Wirkstoff enthältende Beschichtung oder Imprägnierung aufweist. Als Beispiel für ein solches erfindungsgemäßes Implantat sind Platten, Nägel, Schrauben, Hüftgelenke, Katheter oder dergleichen medizintechnische Vorrichtungen zu nennen.

Insbesondere kann das Implantat in Form eines textilen Gebildes, insbesondere eines textilen Flächengebildes für die Gefäßchirurgie vorliegen. In einer bevorzugten Ausführungsform kann das Implantat in Form einer Gefäßprothese vorliegen.
Bei dem Implantat gemäß der Erfindung kann es sich in einer besonders bevorzugten Ausführungsform um eine vorgefertigte Gefäßprothese handeln. Bekannt sind beispielsweise Gefäßprothesen aus biologischem Material wie Bovine Grafts, Nabelschnurvenen oder autologe Venentransplantate. Femer sind Gefäßprothesen aus synthetischen Polymeren üblich, die aus vom Körper nichtresorbierbaren Polymeren, aus resorbierbaren Polymeren oder Kombinationen davon hergestellt sein können. Beispiele für nichtresorbierbare Implantatmaterialien sind Polytetrafluorethylen (PTFE), Polyester wie Polyethylenterephthalat, Polypropylen oder Polyurethane Beispiele für resorbierbare Implantatmaterialien sind Polylactide, Polyglykolide, Copolymere von diesen, Glykolid-Trimethylencarbonatcopolymere, Copolymere mit anderen resorbierbaren cyclischen Estern oder Dioxanonen sowie Terpolymeren davon.

Implantatmaterialien für die Gefäßchirurgie können in Form von textilen und nichttextilen Flächengebilden vorliegen, die entweder auf übliche Weise durch Weben, Wirken oder Stricken gebildet wurden und gegebenenfalls als Velourmaterial ausgebildet sind oder aber durch Sprühen, Tauchen etc. hergestellt wurden. Solche flächigen Materialien können als sogenanntes Patch zum Verschließen von größeren Öffnungen in Gefäßen oder Körpergewebe verwendet werden. Außerdem können aus solchen textilen Flächengebilden schlauchförmige Gefäßprothesen ausgebildet werden. Andererseits können Gefäßprothesen direkt durch Herstellen eines schlauchförmigen Gebildes aus Implantatmaterial, insbesondere durch Wirken oder Stricken gebildet werden. Gegebenenfalls können solche Gefäßprothesen auch verzweigt ausgebildet sein. Eine besonders bevorzugte Ausführungsform von bekannten Gefäßprothesen, hergestellt aus Polyester, ist unter dem Warenzeichen UNI-GRAFT von B. Braun Melsungen im Handel erhältlich.

Bei dem erfindungsgemäßen Implantat kann eine Beschichtung aus mindestens einem antimikrobiotischen Wirkstoff und mindestens einem resorbierbaren Polymer gebildet sein. Das Beschichten kann nach üblichen und den Fachleuten bekannten Beschichtungsverfahren vorgenommen werden, wie beispielsweise Tauch- oder Sprühverfahren. Zu diesem Zweck können der Wirkstoff und das resorbierbare Polymer in einer geeigneten Zusammensetzung, beispielsweise in Form einer Lösung oder einer Suspension aufgebracht werden. Mit Vorteil können der antimikrobiotische Wirkstoff und das Polymer für die Beschichtung im selben Lösemittel gelöst sein. Beispiele für geeignete Lösemittel umfassen Chloroform, Hexafluoraceton und Hexafluorisopropanol.

Erfindungsgemäß ist bevorzugt, dass der antimikrobiotische Wirkstoff in Wasser schwerlöslich ist. Mit Vorteil kann der antimikrobiotische Wirkstoff eine Löslichkeit in Wasser von weniger als 100 mg/ml, insbesondere weniger als 10 mg/ml, bevorzugt weniger als 1 mg/ml aufweisen. Bedingt durch die geringe Löslichkeit des Wirkstoffes in Wasser ergibt sich auch eine verzögerte Lösung des Wirkstoffes in vivo. Dadurch wird der Wirkstoff langsam und über einen längeren Zeitraum in relativ geringen Konzentrationen in den Organismus freigesetzt, so daß sich ein Retardeffekt der Wirkstoff-Freisetzung zeigt. Das Freisetzungsverhalten von antimikrobiellen Wirkstoffen wird nachfolgend in den Beispielen ausführlicher beschrieben.

Beim Implantat gemäß der Erfindung kann der antimikrobiotische Wirkstoff ausgewählt sein aus der Gruppe von gegen Bakterien, Pilze und Viren wirksamen Substanzen oder einer Mischung davon. Erfindungsgemäß zu verwendende antimikrobielle Wirkstoffe richten sich gegen insbesondere für den Menschen pathologische Keime, beispielsweise gegen grampositive Keime wie Staphylococcus aureus, Staphylococcus albus und Streptococcus, gramnegative Keime wie Escherichia coli, Bacterium proteus, Pseudomonas, Enterococcus und Klebsiella. In einer Ausführungsform kann der antimikrobiotische Wirkstoff ein Antibiotikum sein. Zur Prophylaxe und Therapie mit Antibiotika finden im Bereich der Gefäßchirurgie beispielsweise Cefalosporine wie Cefazolin oder Cefamandol, Netilmicin, Penicilline wie Oxacillin oder Mezlocillin, Tetracyclin, Metronidazol oder Aminoglykoside wie Gentamicin oder Neomycin sowie z. B. Rifampicin Anwendung.

In einer bevorzugten Ausführungsform kann der antimikrobiotische Wirkstoff ein Gentamincinderivat, insbesondere Gentamicincrobefat sein. Gentamicincrobefat wird beispielsweise von der Firma Merck im Handel angeboten. Das Implantat zeichnet sich erfindungsgemäß dadurch aus, dass der antimikrobiotische Wirkstoff in einer Konzentration von 0,1 bis 100 mg, insbesondere von 1 bis 10 mg pro Gramm resorbierbares Implantatmaterial vorhanden sein kann.

Bei dem Implantat gemäß der Erfindung kann die Beschichtung mit antimikrobiotischem Wirkstoff und resorbierbarem Polymer in einer Dicke von 0,001 bis 1 mm, insbesondere von 0,01 bis 0,5 mm vorliegen. Mit Vorteil kann sich das erfindungsgemäße Implantat dadurch auszeichnen, dass seine gesamte Oberfläche beschichtet ist.

Gemäß der Erfindung kann die Freisetzung des antimikrobiotischen Wirkstoffes retardiert, aber relativ schneller als der Abbau des resorbierbaren Materials sein, so dass das resorbierbare Material bei Ende der Wirkstoffabgabe noch nicht vollständig resorbiert ist. Der in Retardform vorliegende Wirkstoff kann aus dem resorbierbaren synthetischen Polymerfilm kontrolliert über einen Zeitraum von mehr als 3 Tagen in die Implantatumgebung abgegeben werden bzw. kann über mehr als diesen Zeitraum auf dem Implantat nachgewiesen werden. Insbesondere kann der Zeitraum der Freisetzung der Hauptmenge des antimikrobiotischen Wirkstoffes 3 bis 15 Tage, bevorzugt 5 bis 15 Tage betragen. In besonderen Ausführungsformen kann die retardierte Freisetzung des Wirkstoffes über 20 und bis zu 100 Tagen dauern. Der erreichbare Retardeffekt der Wirkstoff-Freisetzung steht auch in Zusammenhang mit der Resorbierbarkeit und damit der Verweildauer bis zur vollständigen Resorption des Polymers in vivo. Im allgemeinen ist das Polymer beim Ende der Wirkstoffabgabe noch nicht vollständig resorbiert. Bevorzugt kann die Menge des freigesetzten antimikrobiotischen Wirkstoffes in vivo pro Tag 10 µg bis 500 µg pro Gramm Implantatmaterial betragen.

Das erfindungsgemäße Implantat kann sich mit Vorteil dadurch auszeichnen, dass das resorbierbare Material ein Lactid-Glykolid-Copolymer ist. Beispiele für solche resorbierbaren Copolymere sind gebildet aus Poly(D,L-lactid-co-glycolid) mit einem Verhältnis von Lactid : Glycolid von 75 : 25 (unter dem Warenzeichen RESOMER RG 755 von Boehringer Ingelheim erhältlich) oder 50 : 50 (unter dem Warenzeichen RESOMER RG 506 von Boehringer Ingelheim erhältlich). Bevorzugt kann die Resorptionszeit des Materials in vivo 1 Woche bis 52 Wochen, insbesondere 1 Woche bis 12 Wochen betragen. Vorteilhafterweise kann das Polymer in einer Konzentration von 500 bis 5 mg, insbesondere 200 bis 50 mg pro Gramm Implantatmaterial vorhanden sein. Insbesondere kann das Implantat gemäß der Erfindung dadurch gekennzeichnet sein, dass es vollständig aus resorbierbarem Material besteht.

Das erfindungsgemäße Implantat kann in der Gefäßchirurgie zur Vermeidung von postoperativen Infektionen verwendet werden. Mit Vorteil kann sich die Verwendung des Implantats durch retardierte Freisetzung von antimikrobiotischem Wirkstoff auszeichnen.

Ein Verfahren zur Herstellung eines Implantats ist dadurch gekennzeichnet, dass es aus einem nicht resorbierbaren Grundkörper geformt wird, der mit mindestens einer einen antimikrobiotischen Wirkstoff enthaltenden Beschichtung oder Imprägnierung versehen wird, die aus mindestens einem antimikrobiotischen Wirkstoff und mindestens einem resorbierbaren Material gebildet wird, wobei der antimikrobiotische Wirkstoff in Wasser schwerlöslich ist, insbesondere eine Löslichkeit in Wasser von weniger als 10 mg/ml aufweist, wobei das resorbierbare Material ausgewählt ist aus Polylactiden, Polyglykoliden, Copolymeren von diesen, Glykolid-Trimethylencarbonatcopolymeren, Copolymeren mit anderen resorbierbaren cyclischen Estern oder Dioxanonen sowie Terpolymeren davon, und wobei die Löslichkeit des antimikrobiotischen Wirkstoffs und das Degradationsverhalten des resorbierbaren Materials so aufeinander abgestimmt werden, dass der Wirkstoff im wesentlichen während der gesamten Degradationszeit des resorbierbaren Materials abgegeben wird. Beim erfindungsgemäßen Verfahren kann die Freisetzung von antimikrobiotischem Wirkstoff durch Auflösen infolge Diffusion und/oder Freisetzung infolge Abbau des resorbierbaren Materials erfolgen. In einer Ausführungsform kann das Implantat vollständig aus dem antimikrobiotischen Wirkstoff enthaltenden Polymer hergestellt werden. In einer weiteren Ausführungsform des erfindungsgemäßen Verfahrens kann ein vorgefertigtes Implantat mit einer Mischung aus mindestens einem antimikrobiotischen Wirkstoff und mindestens einem resorbierbaren Material beschichtet werden. In einer besonders bevorzugten Ausführungsform des Verfahrens zur Herstellung. eines Implantats gemäß der Erfindung kann eine vorgefertigte Gefäßprothese beschichtet werden.

Mit Vorteil kann sich das erfindungsgemäße Verfahren dadurch auszeichnen, dass die Beschichtung durch Aufsprühen, insbesondere durch wiederholtes Aufsprühen einer Zusammensetzung aus antimikrobiotischem Wirkstoff und resorbierbarem Material vorgenommen wird. Insbesondere kann eine Beschichtungszusammensetzung aufgebracht werden, die antimikrobiotischen Wirkstoff in einer Konzentration von 0,1 bis 2 Gew.-%, bezogen auf die gesamte Zusammensetzung, insbesondere 0,2 bis 1 Gew.-% aufweist und resorbierbares Material in einer Konzentration von 0,1 bis 5 Gew.-%, bezogen auf die gesamte Zusammensetzung, insbesondere von 0,5 bis 1 Gew.-% aufweist.

Im folgenden wird die Erfindung anhand von Beispielen, Vergleichsbeispielen und bevorzugten Ausführungsformen beschrieben. Es wird Bezug genommen auf die begleitenden Figuren in denen:
- Figur 1: die Freisetzung in vitro von Gentamicincrobefat aus Beschichtungen mit Poly(D,L-lactid-co-glycolid) 75/25 und Poly-L-lactid darstellt,
- Figur 2: die kumulierte Freisetzung in vitro von Gentamicincrobefat aus Beschichtungen mit Poly(D,L-lactid-co-glycolid) 75/25 in Abhängigkeit vom Wirkstoffgehalt in der Beschichtung darstellt,
- Figur 3: die kumulierte Freisetzung in vitro von Gentamicincrobefat aus Beschichtungen mit Poly(D,L-lactid-co-glycolid) 50/50 in Abhängigkeit vom Wirkstoffgehalt in der Beschichtung darstellt,
- Figur 4: die kumulierte Freisetzung in vitro von Gentamicincrobefat aus Beschichtungen mit Poly(D,L-lactid-co-glycolid) 50/50 einer Ausführungsform der Erfindung darstellt,
- Figur 5: die mikrobiologische Bestimmung der Freisetzung von Gentamicincrobefat in vivo im Ham (Hund) darstellt,
- Figur 6: die mikrobiologische Bestimmung der Freisetzung von Gentamicincrobefat in vivo im Wundsekret (Hund) darstellt.

### Vergleichsbeispiel 1

### Beschichtung aus resorbierbarem Polymer mit Gentamicinsulfat

Eine vorgefertigte Gefäßprothese, im Handel erhältlich unter dem Warenzeichen UNI-GRAFT, wird in einem Sprühprozeß mit einer Suspension aus 0,1 % Gentamicinsulfat und 0,5 % eines Lactid-Glycolid-Copolymers in Chloroform beschichtet Bei der so ausgerüsteten Gefäßprothese befindet sich der gut wasserlösliche antimikrobiotische Wirkstoff in der zusätzlichen Beschichtungsschicht aus dem resorbierbaren Polymer. Die Freisetzungskinetik des Wirkstoffes wird durch Einlegen von Prothesenstücken definierter Länge bei 37 °C in Phosphatpuffer und Wechsel der Pufferlösung nach 1, 3, 5, 7 und 24 h bestimmt. Die Bestimmung des Gehalts an Gentamicinsulfat in der Pufferlösung wird mikrobiologisch in einem Agardiffusionstest mit S. aureus durchgeführt. Die in der folgenden Tabelle dargestellten Ergebnisse zeigen, dass durch das Einbringen eines wasserlöslichen Wirkstoffes in eine resorbierbare Polymerschicht kein vorteilhafter Retardeffekt erzielt werden kann.

| Sprühzyklen | Freigesetzte Menge Gentamicin (mg/g Prothese) | | | | |
|---|---|---|---|---|---|
| | 1 h | 3 h | 5 h | 7 h | 24 h |
| 20 | 1,7 | 0,1 | n.n. | n.n. | n.n. |
| 40 | 2,8 | 0,8 | 0,2 | n.n. | n.n. |
| n.n. = nicht nachweisbar | | | | | |

### Vergleichsbeispiel 2

### Versiegelung einer Wirkstoffschicht mit einer Schicht aus resorbierbarem Polymer

Eine vorgefertigte Gefäßprothese (UNI-GRAFT) wird in einem Sprühverfahren mit einer 0,1 %igen wäßrigen Gentamicinsulfatlösung beschichtet. Nach Trocknen wird durch Übersprühen mit einer 0,5 %igen Lactid-Glykolid-Copolymer-Lösung in Chloroform die Wirkstoffschicht mit einer Polymerschicht versiegelt. Die Kinetik der Wirkstoff-Freisetzung wird wie in Beispiel 1 bestimmt, die Ergebnisse sind in der folgenden Tabelle angegeben. Auch hier kann bei Verwendung eines wasserlöslichen Wirkstoffes kein vorteilhafter Retardeffekt erzielt werden.

| Sprühzyklen | Freigesetzte Menge Gentamicin (mg/g Prothese) | | | | |
|---|---|---|---|---|---|
| | 1 h | 3 h | 5 h | 7 h | 24 h |
| 10 | 0,7 | 0,1 | n.n. | n.n. | n.n. |
| 20 | 0,9 | 0,2 | n.n. | n.n. | n.n. |
| n.n. = nicht nachweisbar | | | | | |

Die folgenden Beispiele betreffen die retardierte Freisetzung von Wirkstoff aus einer Beschichtung aus resorbierbarem, hydrolytisch abbaubarem Polymer.

### Beispiel 3

Zur Herstellung von Implantaten mit einer Beschichtung aus einem resorbierbaren, hydrolytisch abbaubaren Polymer, die das schwerlösliche Gentamicinsalz Gentamicincrobefat enthält, werden folgende Lösungen vorbereitet:
1 % Poly(D,L-lactid-co-glycolid) 75/25 (im Handel erhältlich als RESOMER RG 755 von Boehringer Ingelheim) und 0,71 % Gentamicincrobefat in Chloroform
1 % Poly-L-lactid (im Handel erhältlich als RESOMER L 210 von Boehringer Ingelheim) und 0,32 % Gentamicincrobefat in Chloroform.

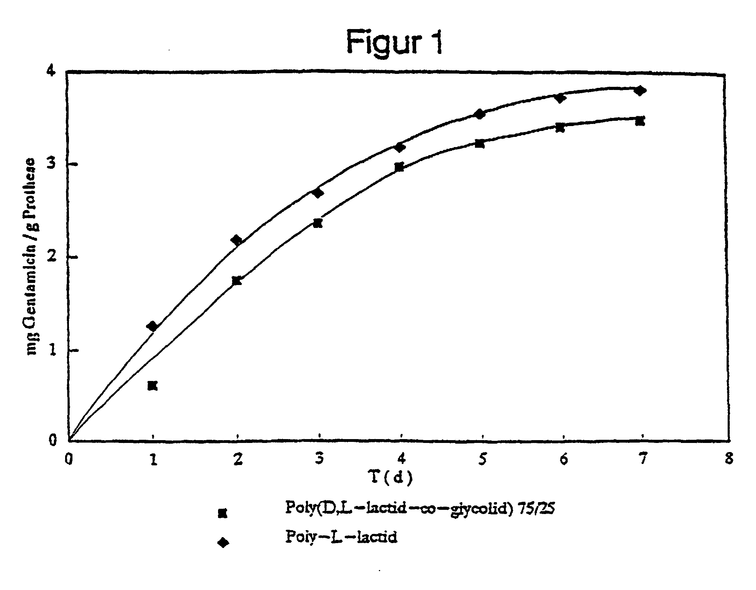

Die Lösungen werden mittels Sprühtechnik mit 40 Sprühzyklen auf vorgefertigte Gefäßprothesen (UNI-GRAFT) aufgebracht, so daß sich eine Polymer/Wirkstoffschicht ausbildet. Die Freisetzungskinetik des Wirkstoffes wird durch Einlegen von Prothesenstücken von definierter Länge und Gewicht in je 5 ml Phosphatpuffer bei 37 °C bestimmt. Das Auslaugmedium wird während des Untersuchungszeitraumes von 7 Tagen alle 24 h gewechselt. Der Gehalt an Gentamicinbase in der Pufferlösung wird mikrobiologisch im Hemmhoftest mit B. subtilis ATCC 6633 im Nachweismedium Antibiotikaagar Nr. 5 (im Handel erhältlich von Merck) nach DIN 58940 bestimmt. Als Referenz wird Gentamicin mit 40 mg/ml biologisch aktivem Gentamicin (Hersteller Merck) betrachtet. Die Versuchsergebnisse sind in der folgenden Tabelle angegeben und in Figur 1 graphisch dargestellt.

Es zeigt sich, dass durch die kombinierte Verwendung eines schwerlöslichen Wirkstoffes und eines resorbierbaren Polymers eine kontrollierte, retardierte Freisetzung des antimikrobiotischen Wirkstoffes erzielt wird.

Nach dem Versuchszeitraum von 7 Tagen wurde ein abgeschnittener Ring der Prothese auf mit B. subtilis beimpften Antibiotikaagar aufgelegt. Für beide Wirkstoff-Formulierungen ergab sich ein deutlicher Hemmhof. Dies zeigt, dass auch nach 7 Tagen noch wirksame Mengen an aktiver Gentamicinbase in der Polymerschicht enthalten sind.

| Polymer | Freigesetzte Menge Gentamicinbase (mg Gentamicinbase/g Prothese) | | | | | | |
|---|---|---|---|---|---|---|---|
| | 1d | 2d | 3d | 4d | 5d | 6d | 7d |
| RESOMER RG 755 | 0,61 | 1,14 | 0,61 | 0,61 | 0,27 | 0,18 | 0,08 |
| RESOMER L 210 | 1,26 | 0,93 | 0,50 | 0,50 | 0,37 | 0,18 | 0,08 |

### Beispiel 4

Zur Herstellung von Implantaten mit einer Beschichtung aus einem resorbierbaren, hydrolytisch abbaubaren Polymer, die das schwerlösliche Gentamicinsalz Gentamicincrobefat enthält, wird eine 0,38 % Gentamicincrobefat und 1 % Poly(D,L-lactid-co-glykolid) 75/25 enthaltende Chloroformlösung hergestellt. Diese Lösung wird bei einem Sprühdruck von 3,5 bar mittels einer Sprühpistole mit einem Düsendurchmesser von 0,3 mm zur Herstellung von vorgefertigten Gefäßprothesen (UNI-GRAFT) mit einer zusätzlichen antimikrobiotischen Wirkstoff enthaltenden Beschichtung versprüht. Durch Variation der durchlaufenen Sprühzyklen werden Prothesen mit einem unterschiedlich dicken Polymerfilm erhalten. Je nach Anzahl der Sprühzyklen variiert somit auch der Wirkstoffgehalt des Polymerfilms und damit der Prothese.

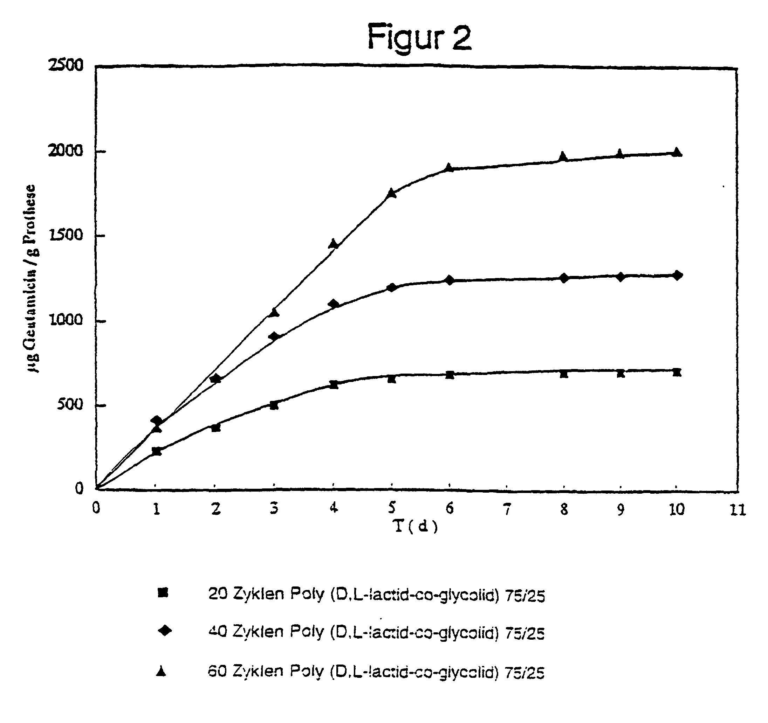

Die Freisetzungskinetik des Wirkstoffes aus den so hergestellten Implantaten wird bestimmt durch Einlegen von Prüfstücken definierter Länge und Gewichtes in je 5 ml Phosphatpuffer (pH 7,4) bei 37 °C. Über einen Versuchszeitraum von 10 Tagen wird die Auslaugflüssigkeit jeweils alle 24 h durch frischen Phosphatpuffer ersetzt. Die Bestimmung der Konzentration an Gentamicinbase in der Auslaugflüssigkeit erfolgt mikrobiologisch nach DIN 58 940 im Hemmhoftest mit S. aureus.
Die Ergebnisse sind in der folgenden Tabelle und in Figur 2 dargestellt.

| Zyklen | Freisetzung von Gentamicinbase (µg Gentamicinbase/g Prothese) | | | | | | | | | |
|---|---|---|---|---|---|---|---|---|---|---|
| | 1d | 2d | 3d | 4d | 5d | 6d | 7d | 8d | 9d | 10d |
| 20 | 231 | 135 | 135 | 118 | 35 | 27 | --- | 13 | 6 | 5 |
| 40 | 414 | 245 | 245 | 195 | 99 | 48 | --- | 18 | 11 | 8 |
| 60 | 368 | 299 | 384 | 406 | 294 | 159 | --- | 72 | 15 | 13 |

### Beispiel 5

Vorgefertigte Gefäßprothesen werden entsprechend Beispiel 4 behandelt mit 0,3 % Gentamicincrobefat und 1 % Poly(D,L-lactid-co-glykolid) 50/50 in Chloroform unter 3,5 bar Sprühdruck und bei einem Düsendurchmesser von 0,3 mm.

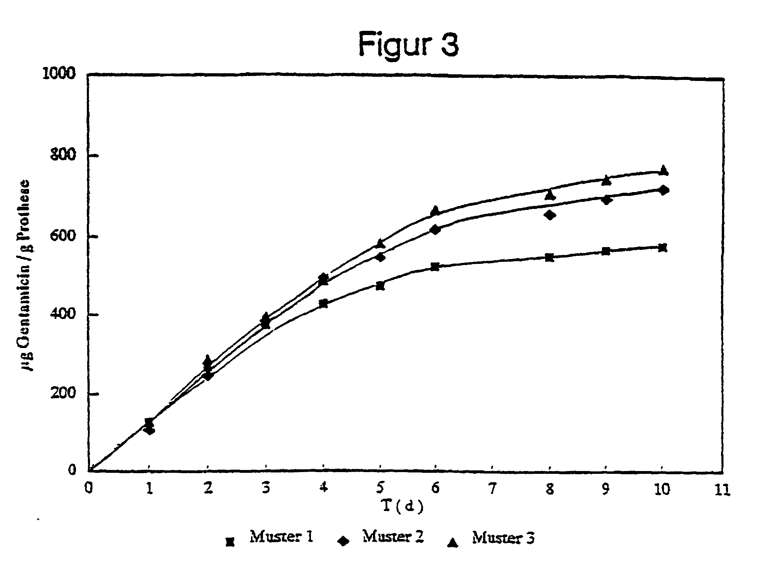

Die Bestimmung der Freisetzungskinetik erfolgt entprechend Beispiel 4. Die Ergebnisse sind in der folgenden Tabelle und in Figur 3 dargestellt.

| Muster Zyklen | | Freisetzung von Gentamicinbase (µg Gentamicinbase/g Prothese) | | | | | | | | | |
|---|---|---|---|---|---|---|---|---|---|---|---|
| | | 1d | 2d | 3d | 4d | 5d | 6d | 7d | 8d | 9d | 10d |
| 1 | 20 | 127 | 143 | 106 | 52 | 45 | 50 | --- | 27 | 17 | 11 |
| 2 | 40 | 107 | 141 | 138 | 107 | 54 | 73 | --- | 39 | 39 | 16 |
| 3 | 60 | 128 | 162 | 107 | 90 | 96 | 86 | --- | 41 | 37 | 26 |

Eine Bestimmung des Restgehaltes an Gentamicinbase auf der Prothese nach Beendigung des Freisetzungsexperiments ergibt die folgende Gesamtbilanz:

| Muster | Gentamicinbase freigesetzt (mg/g Prothese) | Gentamicinbase Restgehalt (mg/g Prothese) | Gentamicinbase Gesamtmenge (mg/g Prothese) |
|---|---|---|---|
| 1 | 0,58 | 0,54 | 1,12 |
| 2 | 0,73 | 1,08 | 1,81 |
| 3 | 0,77 | 2,38 | 3,13 |

### Beispiel 6

### Freisetzung in vitro

Vorgefertigte Gefäßprothesen werden entsprechend Beispiel 4 hergestellt unter folgenden Bedingungen: 0,3 % Gentamicincrobefat, 1 % Poly(D,L-lactidco-glykolid) 50/50. 3,5 bar Sprühdruck, 0,5 mm Düsendurchmesser und 60 Sprühzyklen.

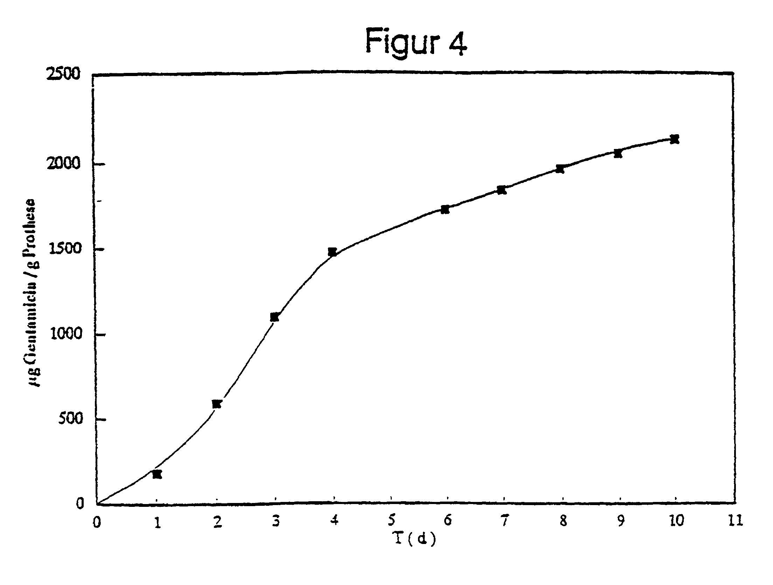

Die Bestimmung der Freisetzungskinetik in vitro erfolgt entsprechend Beispiel 4. Die Ergebnisse sind in der folgenden Tabelle und in Figur 4 dargestellt.

| Freisetzung Gentamicinbase (µg Gentamicinbase/g Prothese) kumuliert | | | | | | | | |
|---|---|---|---|---|---|---|---|---|
| 1d | 2d | 3d | 4d | 6d | 7d | 8d | 9d | 10d |
| 181,0 | 409,8 | 501,6 | 375,7 | 251,7 | 119,3 | 119,3 | 83,4 | 83,4 |

Nach Beendigung des Freisetzungsversuches in vitro wird je ein Prothesenring auf mit S. aureus beimpften Antibiotikaagar aufgelegt. Es ergab sich eine Hemmung von durchschnittlich 31 mm (Bestimmungsgrenze 10 mm). Dies zeigt, dass auch nach 10 Tagen im Freisetzungsversuch noch deutliche Mengen aktiver Gentamicinbase in der Polymerschicht enthalten sind.

Aus der Bestimmung des Gesamtgehaltes an Wirkstoff auf der Prothese (3,5 mg Gentamicinbase/g Prothese) ergibt sich eine Wirkstoff-Freisetzung von insgesamt ca. 60 % nach 10 Tagen.

### Freisetzung in vivo

Zur Untersuchung der Freisetzungskinetik von Gentamicin aus den Implantaten in vivo wurden wirkstoffhaltige Gefäßprothesen durch infrarenalen Aortenersatz im Hund funktionell implantiert. Bestimmt wurde der Antibiotikaspiegel im Serum, Harn und Wundsekret. Der Versuchszeitraum betrug 72 Stunden.

Die Bestimmung der Antibiotikakonzentration im Serum erfolgte mikrobiologisch im Agardiffusionstest. Die Bestimmungsgrenze liegt bei 0,06 µg/ml (Eichkurve Gentamicinsulfat in Serum). Es konnte zu keinem Zeitpunkt im Serum das Antibiotikum nachgewiesen werden.

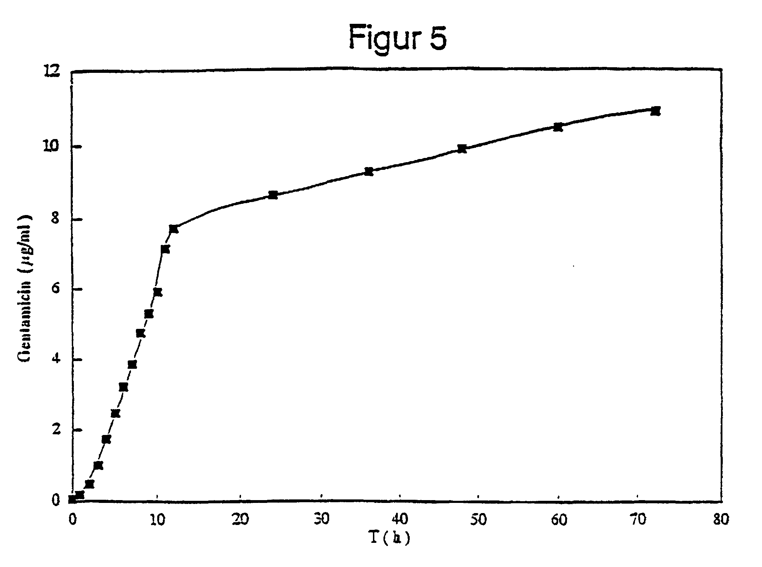

Die Bestimmung der Antibiotikakonzentration im Harn erfolgte mikrobiologisch im Agardiffusionstest. Die Bestimmungsgrenze liegt bei 0,06 µg/ml (Eichkurve Gentamicinsulfat im Harn). Die Ergebnisse sind in der folgenden Tabelle 1 zusammengefaßt und in Figur 5 graphisch dargestellt. Über den gesamten Untersuchungszeitraum konnte im Ham Gentamicin nachgewiesen werden.

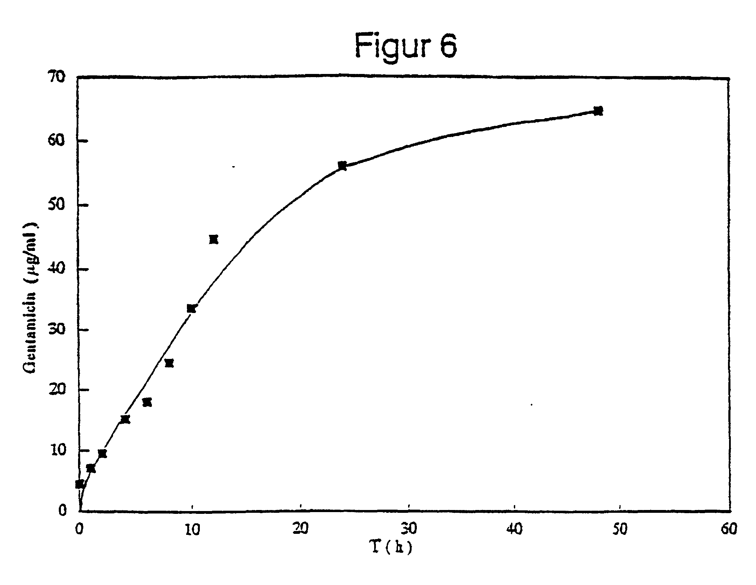

Die Bestimmung der Antibiotikakonzentration im Wundsekret erfolgte mikrobiologisch im Agardiffusionstest. Die Bestimmungsgrenze liegt bei 0,06 µg/ml (Eichkurve Gentamicinsulfat in Serum). Die Ergebnisse sind in Tabelle 1 zusammengefaßt und in Figur 6 graphisch dargestellt. Eine Bestimmung war mangels Wundsekret nur über einen Zeitraum von 48 Stunden möglich. Über den gesamten Untersuchungszeitraum konnte im Wundsekret Gentamicin nachgewiesen werden.

Nach Beendigung des Versuches wurde die Prothese explantiert. Ringe der Prothesen wurden auf mit S. aureus beimpften Antibiotikaagar aufgelegt und der Hemmhof gemessen.

| | | | | |
|---|---|---|---|---|
| Explantation nach | 12 h | 24 h | 48 h | 72 h |
| Hemmhofdurchmesser | 31 mm | 30 mm | 28 mm | 24 mm |

Auch nach 72 h Implantation wird noch eine deutliche Menge aktiver Gentamicinbase auf dem Implantat festgestellt.

**Tabelle 1**

| Mikrobiologische Bestimmung der Freisetzung von Gentamicin kumuliert | | |
|---|---|---|
| Zeit (h) | Harn (µg/ml) | Wundsekret (µg/ml) |
| p. op. | 0,07 | 4,50 |
| 1 | 0,19 | 7,07 |
| 2 | 0,48 | 9,58 |
| 3 | 1,01 | --- |
| 4 | 1,75 | 15,06 |
| 5 | 2,49 | --- |
| 6 | 3,23 | 18,03 |
| 7 | 3,86 | --- |
| 8 | 4,75 | 24,54 |
| 9 | 5,30 | --- |
| 10 | 5,92 | 33,66 |
| 11 | 7,16 | --- |
| 12 | 7,72 | 44,55 |
| 24 | 8,60 | 55,67 |
| 36 | 9,23 | --- |
| 48 | 9,86 | 64,79 |
| 60 | 10,46 | --- |
| 72 | 10,89 | --- |

## Patentansprüche

1. Implantat, das mindestens teilweise und mindestens an den infektionsgeschützten Oberflächenbezirken aus resorbierbarem Material besteht und in diesem einen antimikrobiotischen Wirkstoff enthält, einen nicht resorbierbaren Grundkörper aufweist, der mindestens eine den antimikrobiotischen Wirkstoff enthaltende Beschichtung oder Imprägnierung aufweist, die aus mindestens einem antimikrobiotischen Wirkstoff und mindestens einem resorbierbaren Material gebildet ist, **dadurch gekennzeichnet, dass** der antimikrobiotische Wirkstoff in Wasser schwerlöslich ist, insbesondere eine Löslichkeit in Wasser von weniger als 10 mg/ml aufweist, und die Löslichkeit des antimikrobiotischen Wirkstoffes so gewählt ist, dass er im wesentlichen während der gesamten Degradationszeit des Materials abgegeben wird, wobei das resorbierbare Material ausgewählt ist aus Polylactiden, Polyglykoliden, Copolymeren von diesen, Glykolid-Trimethylencarbonatcopolymeren, Copolymeren mit anderen resorbierbaren cyclischen Estern oder Dioxanonen sowie Terpolymeren davon.

2. Implantat nach Anspruch 1, **dadurch gekennzeichnet, dass** die Löslichkeit des antimikrobiotischen Wirkstoffes und die Degradationszeit des Materials so aufeinander abgestimmt sind, dass der antimikrobiotische Wirkstoff während der Degradationszeit in abnehmenden Mengen abgegeben wird.

3. Implantat nach einem der vorhergehenden Ansprüche, **dadurch gekennzeichnet, dass** es in Form eines textilen Gebildes, insbesondere eines textilen Flächengebildes für die Gefäßchirurgie vorliegt.

4. Implantat nach einem der vorhergehenden Ansprüche, **dadurch gekennzeichnet, dass** es in Form einer Gefäßprothese vorliegt.

5. Implantat nach einem der vorhergehenden Ansprüche, **dadurch gekennzeichnet, dass** der antimikrobiotische Wirkstoff eine Löslichkeit in Wasser von weniger als 1 mg/ml aufweist.

6. Implantat nach einem der vorhergehenden Ansprüche, **dadurch gekennzeichnet, dass** der antimikrobiotische Wirkstoff ausgewählt ist aus der Gruppe von gegen Bakterien, Pilze und Viren wirksamen Substanzen oder einer Mischung davon.

7. Implantat nach einem der vorhergehenden Ansprüche, **dadurch gekennzeichnet, dass** der antimikrobiotische Wirkstoff ein Antibiotikum ist.

8. Implantat nach einem der vorhergehenden Ansprüche, **dadurch gekennzeichnet, dass** der antimikrobiotische Wirkstoff ein Gentamicinderivat, insbesondere Gentamicincrobefat ist.

9. Implantat nach einem der vorhergehenden Ansprüche, **dadurch gekennzeichnet, dass** der antimikrobiotische Wirkstoff in einer Konzentration von 0,1 bis 100 mg, insbesondere von 1 bis 10 mg pro Gramm resorbierbarem Implantatmaterial vorhanden ist.

10. Implantat nach einem der vorhergehenden Ansprüche, **dadurch gekennzeichnet, dass** die Beschichtung mit antimikrobiotischem Wirkstoff und resorbierbarem Material in einer Dicke von 0,001 bis 1 mm, insbesondere von 0,01 bis 0,5 mm vorliegt.

11. Implantat nach einem der vorhergehenden Ansprüche, **dadurch gekennzeichnet, dass** die gesamte Oberfläche des Implantats beschichtet ist.

12. Implantat nach einem der Ansprüche 2 bis 11, **dadurch gekennzeichnet, dass** die Freisetzung des antimikrobiotischen Wirkstoffes retardiert, aber relativ schneller als der Abbau des resorbierbaren Materials ist, so dass das resorbierbare Material bei Ende der Wirkstoffabgabe noch nicht vollständig resorbiert ist.

13. Implantat nach einem der vorhergehenden Ansprüche, **dadurch gekennzeichnet, dass** der Zeitraum der Freisetzung der Hauptmenge des antimikrobiotischen Wirkstoffes 3 bis 15 Tage, insbesondere 5 bis 15 Tage beträgt.

14. Implantat nach einem der vorhergehenden Ansprüche, **dadurch gekennzeichnet, dass** die Menge des freigesetzten antimikrobiotischen Wirkstoffes in vivo pro Tag 10 µg bis 500 µg pro Gramm Implantatmaterial beträgt.

15. Implantat nach einem der vorhergehenden Ansprüche, **dadurch gekennzeichnet, dass** das resorbierbare Material ein Lactid-Glykolid-Copolymer, insbesondere ein Poly(D,L-Lactid-co-Glykolid) ist.

16. Implantat nach einem der vorhergehenden Ansprüche, **dadurch gekennzeichnet, dass** die Resorptionszeit des resorbierbaren Materials in vivo 1 Woche bis 52 Wochen, insbesondere 1 Woche bis 12 Wochen beträgt.

17. Verfahren zur Herstellung eines Implantats, **dadurch gekennzeichnet, dass** es aus einem nicht resorbierbaren Grundkörper geformt wird, der mit mindestens einer einen antimikrobiotischen Wirkstoff enthaltenden Beschichtung oder Imprägnierung versehen wird, die aus mindestens einem antimikrobiotischen Wirkstoff und mindestens einem resorbierbaren Material gebildet wird, wobei der antimikrobiotische Wirkstoff in Wasser schwerlöslich ist, insbesondere eine Löslichkeit in Wasser von weniger als 10 mg/ml aufweist, wobei das resorbierbare Material ausgewählt ist aus Polylactiden, Polyglykoliden, Copolymeren von diesen, Glykolid-Trimethylencarbonatcopolymeren, Copolymeren mit anderen resorbierbaren cyclischen Estern oder Dioxanonen sowie Terpolymeren davon, und wobei die Löslichkeit des antimikrobiotischen Wirkstoffs und das Degradationsverhalten des resorbierbaren Materials so aufeinander abgestimmt werden, dass der Wirkstoff im wesentlichen während der gesamten Degradationszeit des resorbierbaren Materials abgegeben wird.

18. Verfahren nach Anspruch 17, **dadurch gekennzeichnet, dass** ein vorgefertigtes Implantat mit einer Mischung aus mindestens einem antimikrobiotischen Wirkstoff und mindestens einem resorbierbaren Material beschichtet wird.

19. Verfahren nach einem der Ansprüche 17 oder 18, **dadurch gekennzeichnet, dass** eine vorgefertigte Gefäßprothese beschichtet wird.

20. Verfahren nach einem der Ansprüche 17 bis 19, **dadurch gekennzeichnet, dass** die Beschichtung durch Aufsprühen, insbesondere durch wiederholtes Aufsprühen einer Zusammensetzung mit antimikrobiotischem Wirkstoff und resorbierbarem Material vorgenommen wird.

21. Verfahren nach einem der Ansprüche 17 bis 20, **dadurch gekennzeichnet, dass** eine Beschichtungszusammensetzung aufgebracht wird, die antimikrobiotischen Wirkstoff in einer Konzentration von 0,1 bis 2 Gew.-%, bezogen auf die gesamte Zusammensetzung, insbesondere 0,2 bis 1 Gew.-% aufweist und resorbierbares Material in einer Konzentration von 0,1 bis 5 Gew.-%, bezogen auf die gesamte Zusammensetzung, insbesondere von 0,5 bis 1 Gew.-% aufweist.

## Claims

1. An implant at least partly and at least in the infection-protected surface regions, comprises resorbable material and contains therein an antimicrobiotic substance, has a non-resorbable body, which has at least one coating or impregnation containing the antimicrobiotic substance and which is formed from at least one antimicrobiotic substance and at least one resorbable material, **characterized in that** the antimicrobiotic substance is difficultly soluble in water and in particular has a solubility in water of less than 10 mg/ml, and the solubility of the antimicrobiotic substance is chosen in such a way that it is essentially delivered during the entire degradation time of the material, the resorbable material being selected from among polylactides, polyglycolides, copolymers thereof, glycolide-trimethylene carbonate copolymers, copolymers with other resorbable, cyclic esters or dioxanones, as well as terpolymers thereof.

2. Implant according to claim 1, **characterized in that** the solubility of the antimicrobiotic substance and the degradation time of the material are synchronized with one another in such a way that the antimicrobiotic substance is delivered in decreasing quantities during the degradation time.

3. Implant according to one of the preceding claims, **characterized in that** it is in the form of a textile structure, particularly a textile fabric for vascular surgery.

4. Implant according to one of the preceding claims, **characterized in that** it is in the form of a vascular prosthesis.

5. Implant according to one of the preceding claims, **characterized in that** the antimicrobiotic substance has a solubility in water of less than 1 mg/ml.

6. Implant according to one of the preceding claims, **characterized in that** the antimicrobiotic substance is chosen from the group of substances, or a mixture thereof, active against bacteria, fungi and viruses.

7. Implant according to one of the preceding claims, **characterized in that** the microbiotic substance is an antibiotic.

8. Implant according to one of the preceding claims, **characterized in that** the antimicrobiotic substance is a gentamicin derivative, particularly gentamicin crobefate.

9. Implant according to one of the preceding claims, **characterized in that** the antimicrobiotic substance is present in a concentration of 0.1 to 100 mg, particularly 1 to 10 mg per gram of resorbable implant material.

10. Implant according to one of the preceding claims, **characterized in that** the coating with antimicrobiotic substance and resorbable material is present in a thickness of 0.001 to 1 mm, particularly 0.01 to 0.5 mm.

11. Implant according to one of the preceding claims, **characterized in that** the entire implant surface is coated.

12. Implant according to one of the claims 2 to 11, **characterized in that** there is a sustained release of the antimicrobiotic substance, which takes place relatively faster than the decomposition of the resorbable material, so that the latter is still not completely resorbed at the end of active substance delivery.

13. Implant according to one of the preceding claims, **characterized in that** the time period for the release of the main quantity of the antimicrobiotic substance is 3 to 15 days, particularly 5 to 15 days.

14. Implant according to one of the preceding claims, **characterized in that** the quantity of released antimicrobiotic substance in vivo and per day is 10 to 500 æg per gram of implant material.

15. Implant according to one of the preceding claims, **characterized in that** the resorbable material is a lactide-glycolide copolymer, particularly a poly(D,L-lactide-co-glycolide).

16. Implant according to one of the preceding claims, **characterized in that** the resorption time of the resorbable material in vivo is 1 to 52 weeks, particularly 1 to 12 weeks.

17. Method for the manufacture of an implant, **characterized in that** it is formed from a non-resorbable body, which is provided with at least one coating or impregnation containing an antimicrobiotic substance and formed from at least one antimicrobiotic substance and at least one resorbable material, the antimicrobiotic substance being difficultly soluble in water and in particular having a solubility in water of less than 10 mg/ml, the resorbable material being chosen from among polylactides, polyglycolides, copolymers thereof, glycolide-trimethylene carbonate copolymers, copolymers with other resorbable cyclic esters or dioxanones, as well as terpolymers thereof and where the solubility of the antimicrobiotic substance and the degradation of the resorbable material are so synchronized with one another that the active substance is delivered essentially throughout the degradation time of the resorbable material.

18. Method according to claim 17, **characterized in that** a prefabricated implant is coated with a mixture of at least one antimicrobiotic substance and at least one resorbable material.

19. Method according to one of the claims 17 or 18, **characterized in that** a prefabricated vascular prosthesis is coated.

20. Method according to one of the claims 17 to 19, **characterized in that** coating takes place by spraying, particularly by the repeated spraying of a composition with an antimicrobiotic substance and a resorbable material.

21. Method according to one of the claims 17 to 20, **characterized in that** the coating composition is applied having antimicrobiotic substance in a quantity of 0.1 to 2 wt.%, based on the total composition and in particular 0.2 to 1 wt.%, and resorbable material in a concentration of 0.1 to 5 wt.%, based on the total composition and in particular 0.5 to 1 wt.%.

## Revendications

1. Implant qui se compose de matériau résorbable au moins partiellement et au moins aux régions de surface protégées des infections et qui contient dans ce matériau une substance antimicrobiotique qui présente un corps de base non résorbable qui présente au moins un revêtement ou une imprégnation contenant la substance antimicrobiotique, qui est formé à partir d'au moins une substance antimicrobiotique et au moins d'un matériau résorbable, **caractérisé en ce que** la substance antimicrobiotique est difficilement soluble dans l'eau, présente en particulier une solubilité dans l'eau inférieure à 10 mg/ml et **en ce que** la solubilité de la substance antimicrobiotique est choisie de sorte qu'elle puisse être délivrée essentiellement pendant toute la durée de dégradation du matériau, le matériau résorbable étant sélectionné parmi les polylactides, les polyglycolides, leurs copolymères, les copolymères de carbonate de triméthylène et de glycolide, les copolymères avec d'autres esters cycliques résorbables ou des dioxanones ainsi que leurs terpolymères.

2. Implant selon la revendication 1, **caractérisé en ce que** la solubilité de la substance antimicrobiotique et la durée de dégradation du matériau étant coordonnées l'une à l'autre de sorte que la substance antimicrobiotique est délivrée pendant la durée de dégradation en quantités décroissantes.

3. Implant selon l'une des revendications précédentes, **caractérisé en ce qu'**il se trouve sous forme d'une structure textile, en particulier d'une structure textile étendue pour la chirurgie vasculaire.

4. Implant selon l'une des revendications précédentes, en ce qu'il existe sous forme de prothèse vasculaire.

5. Implant selon l'une des revendications précédentes, **caractérisé en ce que** la substance antimicrobiotique présente une solubilité dans l'eau inférieure à 1 mg/ml.

6. Implant selon l'une des revendications précédentes, **caractérisé en ce que** la substance antimicrobiotique est choisie parmi le groupe de substances efficaces contre les bactéries, les fungi et les virus ou leur mélange.

7. Implant selon l'une des revendications précédentes, **caractérisé en ce que** la substance antimicrobiotique est antibiotique.

8. Implant selon l'une des revendications précédentes, **caractérisé en ce que** la substance antimicrobiotique est un dérivé de gentamicine, en particulier crobefat de gentamicine.

9. Implant selon l'une des revendications précédentes, **caractérisé en ce que** la substance antimicrobiotique est présente dans une concentration de 0,1 à 100 mg, en particulier de 1 à 10 mg par gramme de matériau d'implant résorbable.

10. Implant selon l'une des revendications précédentes, **caractérisé en ce que** le revêtement constitué de substance antimicrobiotique et de matériau résorbable se trouve dans une épaisseur comprise entre 0,001 et 1 mm, en particulier 0,01 à 0,5 mm.

11. Implant selon l'une des revendications précédentes, **caractérisé en ce que** la totalité de la surface de l'implant est recouverte.

12. Implant selon l'une des revendications 2 à 11, **caractérisé en ce que** la libération de la substance antimicrobiotique est retardée mais est relativement plus rapide que la dégradation du matériau résorbable de sorte que le matériau résorbable n'est pas encore complètement résorbé à la fin de la délivrance de la substance active.

13. Implant selon l'une des revendications précédentes, **caractérisé en ce que** la période de libération de la quantité principale de la substance antimicrobiotique est de 3 à 15 jours, en particulier 5 à 15 jours.

14. Implant selon l'une des revendications précédentes, **caractérisé en ce que** la quantité de la substance antimicrobiotique libérée in vivo par jour s'élève à 10 µg et 500 µg par gramme.

15. Implant selon l'une des revendications précédentes, **caractérisé en ce que** le matériau résorbable est un copolymère lactide-glycolide, en particulier un Poly(D,L-Lactide-co-Glycolide).

16. Implant selon l'une des revendications précédentes, **caractérisé en ce que** le temps de résorption du matériau résorbable s'élève in vivo de 1 à 52 semaines, en particulier de 1 à 12 semaines.

17. Procédé pour la fabrication d'un implant **caractérisé en ce qu'**il est formé à partir d'un corps de base non résorbable qui est muni d'au moins un revêtement ou imprégnation contenant une substance antimicrobiotique, qui est réalisé au moins à partir d'une substance antimicrobiotique et au moins d'un matériau résorbable, la substance antimicrobiotique étant difficilement soluble dans l'eau, présentant en particulier une solubilité dans l'eau inférieure à 10 mg/ml, le matériau résorbable étant sélectionné parmi les polylactides, les polyglycolides, leurs copolymères, les copolymères de carbonate de triméthylène et de glycolide, les copolymères avec d'autres esters cycliques résorbables ou des dioxanones ainsi que leurs terpolymères, et la solubilité de la substance antimicrobiotique et le comportement de dégradation du matériau résorbable étant coordonnés entre eux de sorte que la substance est délivré essentiellement pendant toute la durée de dégradation du matériau résorbable.

18. Procédé selon la revendication 17, **caractérisé en ce qu'**un implant préfabriqué est revêtu d'un mélange d'au moins une substance antimicrobiotique et d'au moins un matériau résorbable.

19. Procédé selon l'une des revendications 17 ou 18, **caractérisé en ce qu'**une prothèse vasculaire préfabriquée est revêtue.

20. Procédé selon l'une des revendications 17 à 19, **caractérisé en ce que** le revêtement est réalisé par pulvérisation, en particulier par pulvérisation répétée d'une composition d'une substance antimicrobiotique et d'un matériau résorbable.

21. Procédé selon l'une des revendications 17 à 20, **caractérisé en ce qu'**une composition de revêtement est appliquée, laquelle contient la substance antimicrobiotique dans une concentration de 0,1 à 2 pour cent en poids par rapport à la composition totale, en particulier 0,2 à 1 % en poids et le matériau résorbable, dans une concentration de 0,1 à 5 pour cent en poids, par rapport à la composition totale, en particulier de 0,5 à 1 pour cent en poids.
